# EUROPEAN PATENT APPLICATION

(11) **EP 4 563 995 A1**
(43) Date of publication of application: **04.06.2025**
(21) Application number: 23846243.6
(22) Date of filing: 12.07.2023
(51) Int. Cl.: G01N 27/74, C12M 1/34, G01N 33/543

(54) **DETECTION DEVICE AND ANALYSIS DEVICE**

(30) Priority: 27.07.2022 JP 2022119732
(71) Applicant: Blue Industries Inc., Tokyo 130-0013 (JP)
(72) Inventor: KUJI, Tomoaki, Tokyo 130-0013 (JP)
(74) Representative: Rings, Rolf
(86) International application number: PCT/JP2023/025747
(87) International publication number: WO 2024/024512

(57) **Abstract**

[Solution]

A detection device 6 in an embodiment is the detection device 6 that detects an antibody 92 in a sample 9 containing a magnetic insulator 91, and includes a pipe-shaped channel 61 through which the sample 9 flows. The channel 61 includes a main body portion 611 having an insulation property, and a conductor 612 provided at a part of a cross-sectional surface perpendicular to a flow direction. The main body portion 611 is provided with an opposing portion 614 opposed to the conductor 612. A measurement unit 62 that measures at least any of a current and a voltage at the conductor 612, and a temperature control unit 63 that causes a temperature difference between the conductor 612 side of the channel 61 and the opposing portion 614 side of the channel 614 are further provided.

## Description

### TECHNICAL FIELD

The present invention relates to a detection device and an analysis device for detecting a detection target in a liquid sample.

### BACKGROUND ART

Conventionally, a detection method of Patent Document 1 has been disclosed as a technique for detecting a detection target in a liquid sample.

In the detection method of Patent Document 1, by using a channel-type sensor chip in which one substance of two substances specifically binding to one another is fixed as a fixed layer on a wall surface of a channel, which is a microchannel of a channel member through which a liquid sample flows, a liquid sample containing a substance to be detected is made to flow through the channel, a binding substance of an amount corresponding to an amount of the substance to be detected is bound to the fixed layer, then a liquid is moved in the channel at a flow speed with which a shear stress applied to the fixed layer falls within a predetermined range, a non-specifically adsorbed binding substance on the fixed layer is removed thereby, and then a signal from the binding substance on the fixed layer is detected.

Patent Document 1: JP-A-2010-112730

### DISCLOSURE OF THE INVENTION

### PROBLEMS TO BE SOLVED BY THE INVENTION

Now, in the detection method of Patent Document 1, the non-specifically adsorbed binding substance on the fixed layer is removed, and then a signal from the binding substance on the fixed layer is detected. Therefore, there are circumstances in which the flow speed needs to be controlled in the predetermined range and it is difficult to easily detect a detection target in the sample.

Therefore, the present invention has been made in consideration of the above-described circumstances, and it is an object of the present invention to provide a technique that allows facilitating detection of a detection target in a sample.

### SOLUTIONS TO THE PROBLEMS

A detection device according to the present invention is a detection device that detects a detection target in a sample containing a magnetic insulator. The detection device includes a pipe-shaped channel through which the sample flows. The channel includes a main body portion, and a conductor provided at a part of a cross-sectional surface perpendicular to a flow direction. The main body portion is provided with an opposing portion opposed to the conductor. The detection device further includes a measurement unit that measures at least any of a current and a voltage at the conductor, and a temperature control unit that causes a temperature difference between the conductor side of the channel and the opposing portion side of the channel.

An analysis device according to the present invention is an analysis device that includes the detection device of the present invention and analyzes a detection target. The analysis device further includes an evaluation unit that quantifies the detection target based on at least any of a current and a voltage measured by the measurement unit.

### EFFECTS OF THE INVENTION

According to the present invention, a technique that allows facilitating detection of the detection target in the sample can be provided.

### BRIEF DESCRIPTION OF THE DRAWINGS

Fig. 1 is a schematic diagram illustrating an exemplary analysis device in a first embodiment.
Fig. 2 is a diagram illustrating an exemplary detection device.
Fig. 3 is a diagram illustrating an exemplary cross-sectional surface perpendicular to a flow direction of a channel of the detection device.
Fig. 4 is a diagram illustrating an exemplary detection device, Fig. 4A is a diagram illustrating a cross-sectional surface perpendicular to a flow direction of a channel passing through a conductor, and Fig. 4B is a diagram illustrating a cross-sectional surface perpendicular to a flow direction of a channel passing through a magnet.
Fig. 5 is a diagram illustrating an exemplary relation between the antibody content and the current and voltage.
Fig. 6 is a diagram illustrating an exemplary detection method of a detection target in a sample using the detection device, Fig. 6A illustrates the state before a current is generated at the conductor, and Fig. 6B illustrates the state after the current is generated at the conductor.
Fig. 7 is a diagram illustrating an exemplary detection device in a first modification.
Fig. 8 is a diagram illustrating an exemplary cross-sectional surface perpendicular to a flow direction of a channel of the detection device in the first modification.
Fig. 9 is a diagram illustrating an exemplary detection device in the first modification, Fig. 9A is a diagram illustrating a cross-sectional surface perpendicular to a flow direction of a channel passing through a conductor, and Fig. 9B is a diagram illustrating a cross-sectional surface perpendicular to a flow direction of a channel passing through a magnet.
Fig. 10 is a diagram illustrating an exemplary detection method of a detection target in a sample using the detection device in the first modification, Fig. 10A illustrates the state before a current is generated at the conductor, and Fig. 10B illustrates the state after the current is generated at the conductor.

### DESCRIPTION OF PREFERRED EMBODIMENTS

The following describes embodiments for embodying detection and analysis devices to which the present invention is applied in detail with reference to the drawings.

### <First Embodiment: Analysis Device 100>

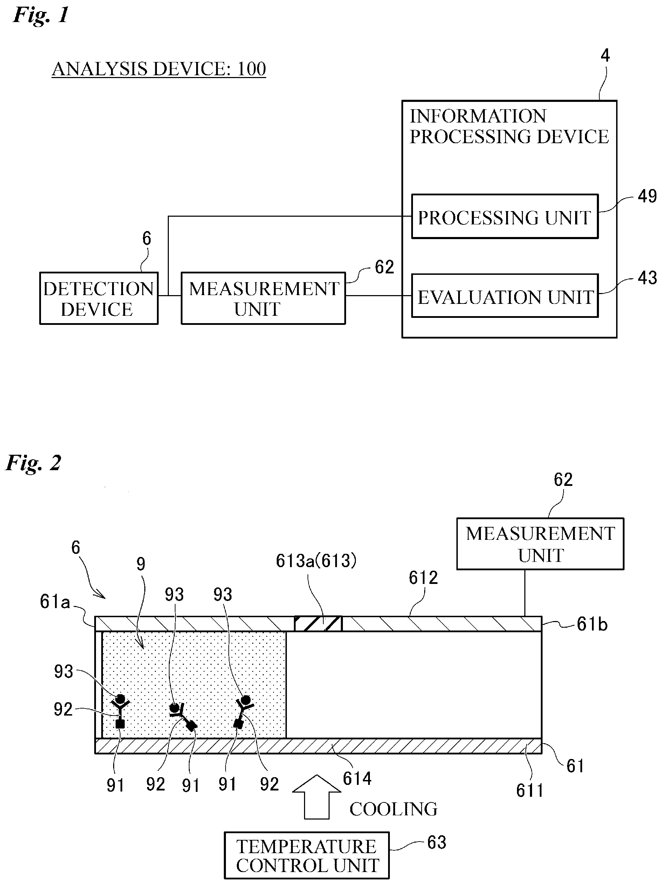

As illustrated in Fig. 1, an analysis device 100 includes a detection device 6, a measurement unit 62, and an information processing device 4. The analysis device 100 analyzes a sample 9 containing a magnetic insulator 91. For the magnetic insulator 91, for example, yttrium iron garnet is used.

### <Detection Device 6>

Fig. 2 is a diagram illustrating an exemplary detection device 6. As illustrated in Fig. 2, the detection device 6 detects a detection target in the sample 9 containing the magnetic insulator 91. The detection device 6 includes a channel 61, the measurement unit 62, and a temperature control unit 63.

### <<Channel 61>>

The channel 61 is formed in a pipe shape, and the sample 9 flows through the channel 61. The channel 61 includes a main body portion 611, a conductor 612, and a magnet 613. The channel 61 may be provided with a flow rate controller (not illustrated) that controls the flow speed and the timing of flowing of the sample 9 that flows. The flow rate controller controls the flow speed, the timing of flowing, and the like of the sample 9 that flows through the channel 61 by a processing unit 49. The channel 61 includes an injection port 61a for injecting the sample 9 at one end side, and a discharge port 61b for discharging the sample 9 at the other end side.

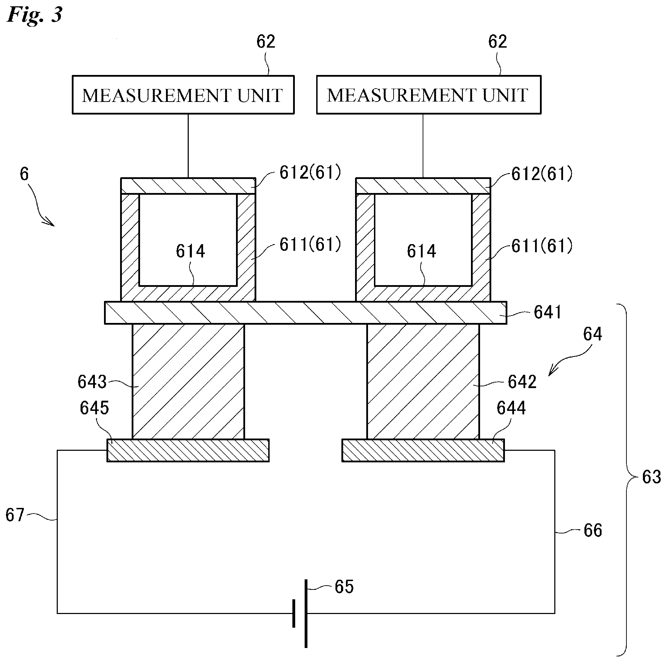

As illustrated in Fig. 3, an insulator of, for example, silicon is used for the main body portion 611, and the main body portion 611 has an insulation property. The main body portion 611 is formed, for example, in a U shape at a cross-sectional surface perpendicular to a flow direction of the channel 61. The main body portion 611 is provided with the conductor 612 at a part of the cross-sectional surface perpendicular to the flow direction of the channel 61, and an opposing portion 614 formed opposed to the conductor 612.

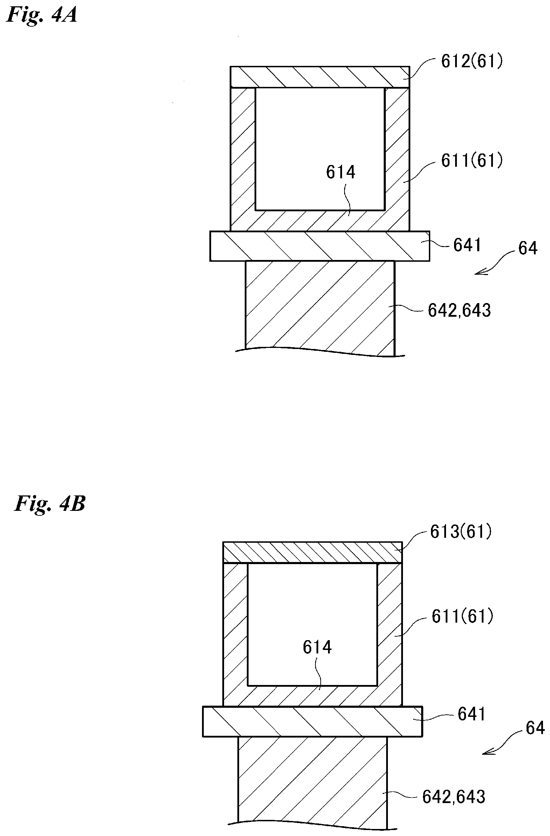

As illustrated in Fig. 4A, the conductor 612 is provided at the main body portion 611 of the channel 61. The conductor 612 has a conductive property, and for example, platinum is used for the conductor 612. The conductor 612 is formed in a plate shape.

As illustrated in Fig. 4B, the magnet 613 is provided at a part of the cross-sectional surface perpendicular to the flow direction of the channel 61 at the conductor 612 side of the channel 61. The magnet 613 is provided in contact with the conductor 612. For the magnet 613, for example, an electromagnet 613a is used. The electromagnet 613a is connected to a current controller (not illustrated). The electromagnet 613a can attract and/or attach the sample containing the magnetic insulator to the electromagnet 613a when the current controller causes a current to flow. The electromagnet 613a can detach the sample attached by the electromagnet 613a from the electromagnet 613a when the current by the current controller does not flow.

### <<Measurement Unit 62>>

As illustrated in Fig. 2, the measurement unit 62 is electrically connected to the conductor 612, and measures at least any of a current and a voltage generated at the conductor 612. For the measurement unit 62, a known device configured to measure at least any of the current and the voltage is used.

### <<Temperature Control Unit 63>>

The temperature control unit 63 causes a temperature difference between the conductor 612 side of the channel 61 and the opposing portion 614 side of the channel 61. At this time, a temperature difference is generated in the magnetic insulator 91 contained in the sample 9 inside the channel 61. The temperature difference causes a spin current to flow through the magnetic insulator 91, and the spin current flows into the conductor 612. This generates a current at the conductor 612, thus causing the spin Seebeck effect. By measuring at least any of the current generated at the conductor 612 and a voltage accompanying this current by the measurement unit 62, the presence/absence of the detection target contained in the sample 9 can be detected.

The temperature control unit 63, for example, cools the opposing portion 614 side of the channel 61, thereby performing control such that the temperature at the magnet 613 side of the channel 61 becomes higher than the temperature at the opposing portion 614 side of the channel 61.

As illustrated in Fig. 3, the temperature control unit 63 includes a Peltier element 64, a current generator 65, a first wiring 66, and a second wiring 67.

As illustrated in Fig. 4B, the Peltier element 64 is provided in contact with the opposite side of the conductor 612 and the magnet 613 across the inside of the channel 61. For the Peltier element 64, a known Peltier element is used. As illustrated in Fig. 3, the Peltier element 64 includes a first electrode portion 641, an N-type semiconductor 642, a P-type semiconductor 643, a second electrode portion 644, and a third electrode portion 645.

For the first electrode portion 641, for example, a metal, such as platinum and copper, is used. As illustrated in Fig. 4B, the first electrode portion 641 is in contact with the opposing portion 614 having an insulation property. The first electrode portion 641 is in contact with the main body portion 611 of the channel 61 on the opposite side of the conductor 612 and the magnet 613 across the inside of the channel 61. The first electrode portion 641 may be a metal provided with a substrate and the like.

As illustrated in Fig. 3, for the N-type semiconductor 642 and the P-type semiconductor 643, known semiconductors are used. The N-type semiconductor 642 and the P-type semiconductor 643 are each provided at the first electrode portion 641 away from one another.

For the second electrode portion 644, for example, a metal, such as platinum and copper, is used. The second electrode portion 644 is provided on the opposite side of the first electrode portion 641 across the N-type semiconductor 642. A metal constituting the second electrode portion 644 may be provided with a substrate of ceramic or the like.

For the third electrode portion 645, for example, a metal, such as platinum and copper, is used. The third electrode portion 645 is provided on the opposite side of the first electrode portion 641 across the P-type semiconductor 643. A metal constituting the third electrode portion 645 may be provided with a substrate of ceramic or the like.

The current generator 65 provides a current to the Peltier element 64. The current generator 65 has a positive side electrically connected to the second electrode portion 644 via the first wiring 66. The current generator 65 has a negative side electrically connected to the third electrode portion 645 via the second wiring 67.

In the current generator 65, when the current is provided to the Peltier element 64, heat is absorbed at the first electrode portion 641 side, and heat is released at the second electrode portion 644 side and the third electrode portion 645 side. Since the first electrode portion 641 is in contact with the opposing portion 614, the temperature control unit 63 can cool the opposing portion 614 side of the channel 61 with the cooled first electrode portion 641 side. As a result, the temperature control unit 63 can perform the control such that the temperature at the magnet 613 side of the channel 61 becomes higher than the temperature at the opposing portion 614 side of the channel 61.

### <Information Processing Device 4>

For the information processing device 4, for example, a personal computer is used. Further, the information processing device 4 may further include an input/output unit that inputs and outputs various kinds of information and a storage unit that stores the various kinds of information.

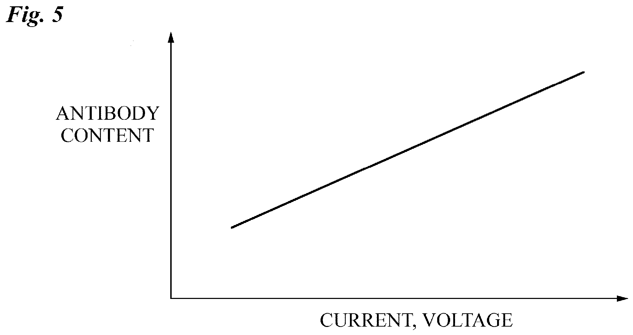

The information processing device 4 includes, for example, an evaluation unit 43. The information processing device 4 stores, for example, a relation between at least any of a current and a voltage preliminarily measured by the measurement unit 62 and the detection target content in the sample 9. For example, as illustrated in Fig. 5, the information processing device 4 stores a relation between at least any of the current and the voltage measured by the measurement unit 62 and the content of an antibody that is the detection target in the sample 9. The information processing device 4 can control, for example, the flow speed and the timing of flowing of the sample 9 that flows through the channel 61 by the processing unit 49.

### <<Evaluation Unit 43>>

The evaluation unit 43 quantifies the detection target content in the sample 9 based on at least any of the current and the voltage measured by the measurement unit 62. For example, the evaluation unit 43 refers to the relation between at least any of the current and the voltage measured by the measurement unit 62 and the detection target content in the sample 9 stored in the information processing device 4, and quantifies the detection target content in the sample 9 based on at least any of the current and the voltage measured by the measurement unit 62. This allows facilitating quantification of the detection target content in the sample 9. For example, the contents of an antibody, an antigen, an enzyme, a substrate, and the like contained in the sample 9 can be quantified.

### (Exemplary Detection Method of Detection Target in Sample 9 Using Detection Device 6)

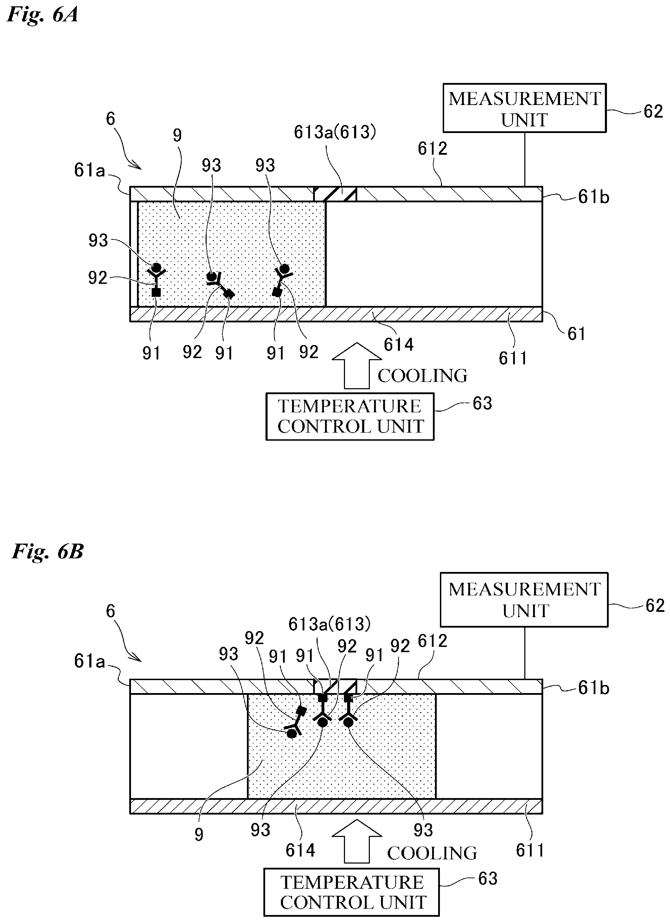

Next, an exemplary detection method of the detection target in the sample 9 using the detection device 6 will be described. Fig. 6 is a diagram illustrating the exemplary detection method of the detection target in the sample 9 using the detection device 6. The sample 9 contains the magnetic insulator 91. The magnetic insulator 91 is bound to an antibody 92 via a binder, such as streptavidin. The antibody 92 is bound to an antigen 93. In the following example, the antibody 92 is assumed as the detection target. The detection target may be the antigen 93. The detection target may be an enzyme or a substrate.

As illustrated in Fig. 6A, the sample 9 is made to flow through the channel 61. At this time, the temperature control unit 63 cools the opposing portion 614 side of the channel 61, thereby performing the control such that the temperature at the conductor 612 side of the channel 61 becomes higher than the temperature at the opposing portion 614 side of the channel 61. This causes the temperature difference between the conductor 612 side of the channel 61 and the opposing portion 614 side of the channel 61.

As illustrated in Fig. 6B, when the sample 9 reaches the conductor 612 and the electromagnet 613a, the sample 9 is stopped inside the channel 61. This causes the magnetic insulator 91 contained in the sample 9 to be attracted to the vicinity of the electromagnet 613a, and attached.

Since the temperature difference has been generated between the conductor 612 side of the channel 61 and the opposing portion 614 side of the channel 61, a temperature difference is generated in the magnetic insulator 91 contained in the sample 9. The temperature difference causes a spin current to flow through the magnetic insulator 91, and the spin current flows into the conductor 612. This generates a current at the conductor 612, thus causing the spin Seebeck effect.

Then, in a state where the sample 9 is stopped inside the channel 61, the current generated at the conductor 612 is measured by the measurement unit 62. Since the sample 9 contains the antibody 92 that is the detection target, the antibody 92 can be detected as the detection target with the current measured by the measurement unit 62. The measurement unit 62 may measure a voltage accompanying the current generated at the conductor 612.

### (Exemplary Quantification Method of Detection Target in Sample 9 Using Analysis Device 100)

Next, an exemplary quantification method of the detection target in the sample 9 using the analysis device 100 in the first embodiment will be described.

By performing a procedure similar to the above-described detection method, at least any of the current and the voltage generated at the conductor 612 is measured by the measurement unit 62. After the measurement by the measurement unit 62, for example, the evaluation unit 43 refers to the relation between at least any of the current and the voltage measured by the measurement unit 62 and the detection target content in the sample 9 stored in the information processing device 4, and quantifies the detection target content in the sample 9 based on at least any of the current and the voltage measured by the measurement unit 62. This allows facilitating quantification of the detection target content in the sample 9.

According to the embodiment, the channel 61 includes the main body portion 611 having an insulation property and the conductor 612 provided at a part of the cross-sectional surface perpendicular to the flow direction, the main body portion 611 is provided with the opposing portion 614 opposed to the conductor 612, and the measurement unit 62 that measures at least any of the current and the voltage at the conductor 612 and the temperature control unit 63 that causes the temperature difference between the conductor 612 side of the channel 61 and the opposing portion 614 side of the channel 61 are provided. This causes the temperature difference in the magnetic insulator 91 contained in the sample 9 inside the channel 61. The temperature difference causes a spin current to flow through the magnetic insulator 91, and the spin current flows into the conductor 612. This generates a current at the conductor 612, thus causing the spin Seebeck effect. By measuring at least any of the current generated at the conductor 612 and the voltage accompanying this current by the measurement unit 62, the presence/absence of the detection target contained in the sample 9 can be detected. As a result, the detection target in the sample 9 can be easily detected.

According to the embodiment, the evaluation unit 43 that quantifies the detection target based on at least any of the current and the voltage measured by the measurement unit 62 is further provided. This allows facilitating quantification of the detection target in the sample 9.

According to the embodiment, the magnet 613 is provided at the conductor 612. This allows attracting and/or attaching the magnetic insulator 91 contained in the sample 9 to the magnet 613. Therefore, the spin current flowing into the conductor 612 can be increased, and the current generated at the conductor 612 can be increased. Accordingly, the detection accuracy of the detection target contained in the sample 9 can be further improved. When the detection target is quantified, the quantification accuracy of the detection target can be further improved.

According to the embodiment, in the state where the sample 9 is stopped inside the channel 61, the measurement unit 62 measures at least any of the current and the voltage generated at the conductor 612 with the measurement unit 62. This allows stabilizing the current generated at the conductor 612. Therefore, the detection accuracy of the detection target can be further improved. When the detection target is quantified, the quantification accuracy of the detection target can be further improved.

According to the embodiment, the magnet 613 is the electromagnet 613a. This facilitates a recovery operation of the sample 9 containing the magnetic insulator 91 attached to the electromagnet 613a after the end of the measurement by the measurement unit 62. Therefore, repeatedly performing the detection and the quantification of the detection target is facilitated.

According to the embodiment, the temperature control unit 63 includes the Peltier element 64 and the current generator 65 that provides a current to the Peltier element 64, and the opposing portion 614 is provided in contact with the electrode portion (for example, the first electrode portion 641) of the Peltier element 64. This allows efficiently performing cooling or heat generation of the opposing portion 614 when a current is provided to the Peltier element 64 and the electrode portion absorbs heat or generates heat. Therefore, the temperature difference between the conductor 612 side of the channel 61 and the opposing portion 614 side of the channel 61 is easily generated.

According to the embodiment, the sample 9 contains the antigen 93 and the antibody 92. This allows facilitating detection of the detection target in a biomaterial that exhibits an antigen-antibody reaction.

While the temperature control unit 63 cools the opposing portion 614 side in the embodiment, the conductor 612 side may be heated in the present invention. Further, in the present invention, the temperature control unit 63 may perform any one of cooling and heating of the opposing portion 614 side, and may perform the other of cooling and heating of the conductor 612 side.

### <Second Embodiment: Analysis Device 100>

The analysis device 100 includes a detection device 6 in a first modification, the measurement unit 62, and the information processing device 4. The analysis device 100 analyzes the sample 9 containing the magnetic insulator 91.

### <First Modification of Detection Device 6>

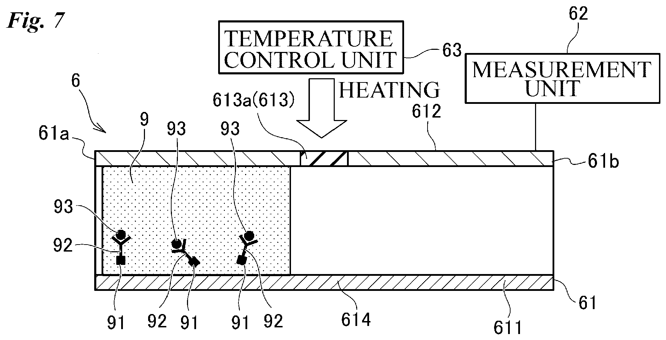

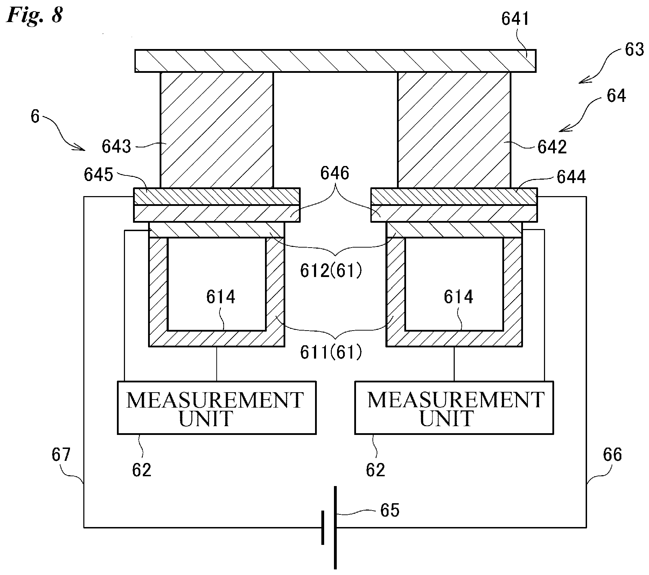

As illustrated in Fig. 7 and Fig. 8, the temperature control unit 63, for example, heats the conductor 612 side of the channel 61, thereby performing the control such that the temperature at the conductor 612 side of the channel 61 becomes higher than the temperature at the opposing portion 614 side of the channel 61. This causes the temperature difference between the conductor 612 side of the channel 61 and the opposing portion 614 side of the channel 61.

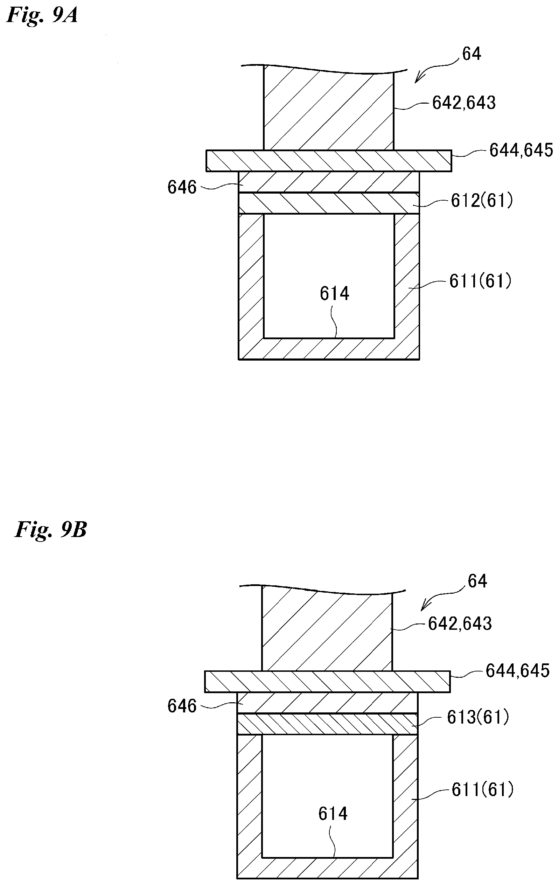

As illustrated in Fig. 9A and Fig. 9B, the second electrode portion 644 is provided at the conductor 612 side of the channel 61 away from the conductor 612. An insulator 646 is provided between the second electrode portion 644 and the conductor 612. This allows preventing a current generated at the conductor 612 from flowing through the second electrode portion 644 when a spin current flows through the conductor 612 and the current is generated. Further, the insulator 646 is provided between the second electrode portion 644 and the magnet 613.

Similarly, the third electrode portion 645 is provided at the conductor 612 side of the channel 61. The insulator 646 is provided between the third electrode portion 645 and the conductor 612. This allows preventing a current generated at the conductor 612 from flowing through the third electrode portion 645 when a spin current flows through the conductor 612 and the current is generated. Further, the insulator 646 is provided between the third electrode portion 645 and the magnet 613.

### (Exemplary Detection Method of Detection Target in Sample 9 by Detection Device 6)

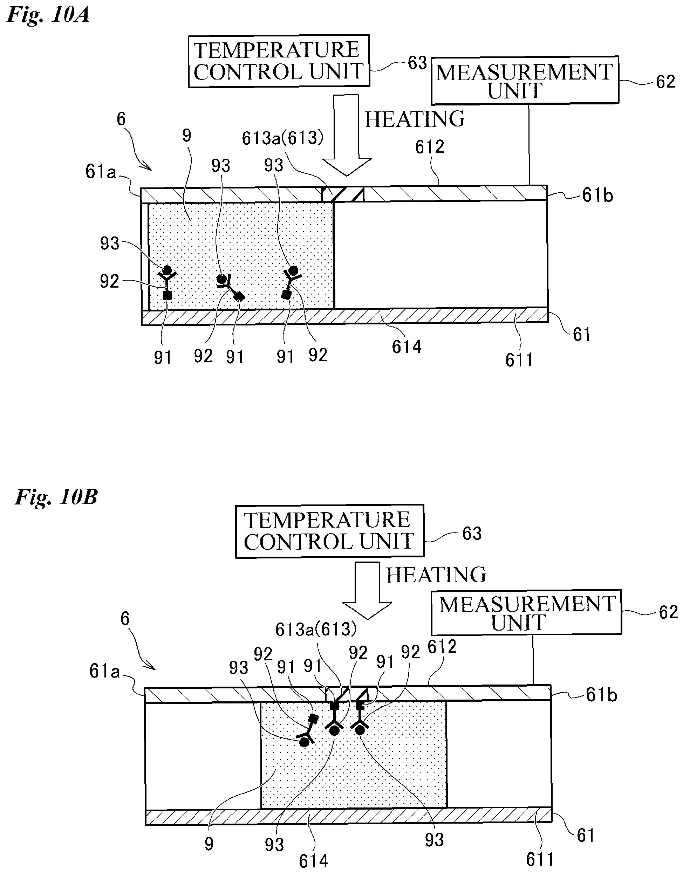

Next, an exemplary detection method of the detection target in the sample 9 by the detection device 6 will be described. Fig. 10 is a diagram illustrating the exemplary detection method of the detection target in the sample 9 by the detection device 6. The sample 9 contains the magnetic insulator 91. The magnetic insulator 91 contains the antibody 92 and the antigen 93 bound via a binder. In the following example, the antibody 92 is assumed as the detection target.

As illustrated in Fig. 10A, the sample 9 is made to flow through the channel 61. At this time, the temperature control unit 63 heats the conductor 612 side of the channel 61, thereby performing the control such that the temperature at the conductor 612 side of the channel 61 becomes higher than the temperature at the opposing portion 614 side of the channel 61. This causes the temperature difference between the conductor 612 side of the channel 61 and the opposing portion 614 side of the channel 61.

As illustrated in Fig. 10B, when the sample 9 reaches the conductor 612 and the electromagnet 613a, the flow of the sample 9 is not stopped, and the sample 9 is kept flowing inside the channel 61. Since the temperature difference is generated between the conductor 612 side of the channel 61 and the opposing portion 614 side of the channel 61, the temperature difference is generated in the magnetic insulator 91 contained in the sample 9 even in this case. The temperature difference causes a spin current to flow through the magnetic insulator 91, and the spin current flows into the conductor 612. This generates a current at the conductor 612, thus causing the spin Seebeck effect. By measuring at least any of the current generated at the conductor 612 and the voltage accompanying this current by the measurement unit 62, the presence/absence of the detection target contained in the sample 9 can be detected.

Since the electromagnet 613a is provided at the conductor 612 side of the channel 61, the magnetic insulator 91 contained in the sample 9 is attracted and/or attached to the electromagnet 613a. Therefore, the spin current flowing into the conductor 612 can be increased, and the current generated at the conductor 612 can be increased.

Further, since the temperature at the conductor 612 side of the channel 61 becomes higher than the temperature at the opposing portion 614 side of the channel 61, by the sample 9 flowing, the flow speed of the sample 9 at the conductor 612 side of the channel 61 becomes higher than the flow speed of the sample 9 at the opposing portion 614 side of the channel 61. This relatively increases the sample 9 flowing through the conductor 612 side of the channel 61. Therefore, the spin current flowing into the conductor 612 can be increased, and the current generated at the conductor 612 can be increased.

Then, in a state where the sample 9 flows, the current generated at the conductor 612 is measured by the measurement unit 62. Since the sample 9 contains the antibody 92 that is the detection target, the antibody 92 can be detected as the detection target with the current measured by the measurement unit 62. The measurement unit 62 may measure a voltage accompanying the current generated at the conductor 612.

When the detection target is quantified, after the measurement by the measurement unit 62, for example, the evaluation unit 43 refers to the relation between at least any of the current and the voltage measured by the measurement unit 62 and the detection target content in the sample 9 stored in the information processing device 4, and quantifies the detection target content in the sample 9 based on at least any of the current and the voltage measured by the measurement unit 62. This allows facilitating quantification of the detection target content in the sample 9.

Since the measurement is performed while the sample 9 flows as described above, the detection and the quantification of the detection target in the sample 9 can be performed at a higher speed.

According to the embodiment, in the state where the sample 9 flows inside the channel 61, the measurement unit 62 measures at least any of the current and the voltage generated at the conductor 612 with the measurement unit 62. Therefore, by sequentially making the sample 9 flow through the channel 61, the detection and the quantification of the detection target in the sample 9 can be performed at a higher speed. Accordingly, the detection target in the sample 9 can be detected in a shorter time. When the detection target is quantified, the detection target can be quantified in a shorter time.

According to the embodiment, the temperature control unit 63 performs the control such that the temperature at the conductor 612 side of the channel 61 becomes higher than the temperature at the opposing portion 614 side of the channel 61, and the measurement unit 62 measures at least any of the current and the voltage generated at the conductor 612 in the state where the sample 9 flows inside the channel 61 with the measurement unit 62. Therefore, the flow speed of the sample 9 at the conductor 612 side of the channel 61 becomes higher than the flow speed of the sample 9 at the opposing portion 614 side of the channel 61. The sample 9 flowing through the conductor 612 side of the channel 61 relatively increases. Therefore, the spin current flowing into the conductor 612 can be increased, and the current generated at the conductor 612 can also be increased. Consequently, the detection accuracy of the detection target contained in the sample 9 can be improved. When the detection target is quantified, the quantification accuracy of the detection target can be improved.

According to the embodiment, the temperature control unit 63 includes the Peltier element 64 and the current generator 65 that provides a current to the Peltier element 64, the conductor 612 is provided away from the electrode portion (for example, the second electrode portion 644 and the third electrode portion 645) of the Peltier element 64, and the insulator 646 is provided between the conductor 612 and the electrode portion of the Peltier element 64. This allows preventing the current generated at the conductor 612 from flowing through the electrode portion of the Peltier element 64. Therefore, the measurement accuracy of at least any of the current and the voltage measured by the measurement unit 62 can be improved. Consequently, the detection accuracy of the detection target contained in the sample 9 can be improved. When the detection target is quantified, the quantification accuracy of the detection target can be improved.

While the embodiments of the present invention have been described above, these embodiments have been presented by way of example, and are not intended to limit the scope of the invention. These embodiments can be embodied in combination as necessary. Further, the present invention can be embodied in various novel embodiments in addition to the above-described embodiments. Therefore, for each of the above-described embodiments, various omissions, substitutions, and changes can be made without departing from the gist of the invention. Such novel embodiments and modifications are included in the scope and the gist of the invention, and are included in the invention described in the claims and the scope of the equivalents of the invention described in the claims.

### DESCRIPTION OF REFERENCE SIGNS

- 100:: Analysis device
- 6:: Detection device
- 61:: Channel
- 611:: Main body portion
- 612:: Conductor
- 613:: Magnet
- 613a:: Electromagnet
- 614:: Opposing portion
- 62:: Measurement unit
- 63:: Temperature control unit
- 64:: Peltier element
- 641:: First electrode portion
- 642:: N-type semiconductor
- 643:: P-type semiconductor
- 644:: Second electrode portion
- 645:: Third electrode portion
- 646:: Insulator
- 65:: Current generator
- 66:: First wiring
- 67:: Second wiring
- 9:: Sample
- 91:: Magnetic insulator
- 92:: Antibody
- 93:: Antigen

## Claims

1. A detection device that detects a detection target in a sample containing a magnetic insulator, comprising
a pipe-shaped channel through which the sample flows, wherein
the channel includes:
a main body portion having an insulation property; and
a conductor provided at a part of a cross-sectional surface perpendicular to a flow direction,
the main body portion is provided with an opposing portion opposed to the conductor, and
the detection device further includes:
a measurement unit that measures at least any of a current and a voltage at the conductor; and
a temperature control unit that causes a temperature difference between the conductor side of the channel and the opposing portion side of the channel.

2. The detection device according to claim 1, wherein
the channel includes a magnet provided at the conductor.

3. The detection device according to claim 2, wherein
the magnet is an electromagnet.

4. The detection device according to claim 1, wherein
the measurement unit measures at least any of the current and the voltage generated at the conductor in a state where the sample is stopped inside the channel.

5. The detection device according to claim 1, wherein
the measurement unit measures at least any of the current and the voltage generated at the conductor in a state where the sample flows inside the channel.

6. The detection device according to claim 4, wherein
the temperature control unit performs control such that a temperature at the conductor side of the channel becomes higher than a temperature at the opposing portion side of the channel.

7. The detection device according to claim 1, wherein
the temperature control unit includes a Peltier element and a current generator that provides a current to the Peltier element, and
the opposing portion is provided in contact with an electrode portion of the Peltier element.

8. The detection device according to claim 1, wherein
the temperature control unit includes a Peltier element and a current generator that provides a current to the Peltier element,
the conductor is provided apart from an electrode portion of the Peltier element, and
an insulator is provided between the conductor and the electrode portion of the Peltier element.

9. The detection device according to claim 1, wherein
the sample contains an antigen and an antibody.

10. An analysis device that includes the detection device according to claim 1 and analyzes a detection target, the analysis device further comprising
an evaluation unit that quantifies the detection target based on at least any of a current and a voltage measured by the measurement unit.
